# EUROPEAN PATENT APPLICATION

(11) **EP 4 393 431 A1**
(43) Date of publication of application: **03.07.2024**
(21) Application number: 23219807.7
(22) Date of filing: 22.12.2023
(51) Int. Cl.: A61B 18/14

(54) **SYSTEMS AND METHODS FOR LINEAR SPINES FORMING A SPHERICAL BASKET FOR IMPROVED TISSUE CONTACT AND CURRENT DELIVERY**

(30) Priority: 29.12.2022 US 202218148400
(71) Applicant: Biosense Webster (Israel) Ltd., 2066717 Yokneam (IL)
(72) Inventor: BEECKLER, Christopher Thomas, Irvine, 92618 (US); KHAN, Ishan, Irvine, 92618 (US); OKARSKI, Kevin Mark, Irvine, 92618 (US)
(74) Representative: Carpmaels & Ransford LLP

(57) **Abstract**

The disclosed technology includes a medical probe comprising a tubular shaft extending along a longitudinal axis and including a proximal end and a distal end. The medical probe further comprises an expandable basket assembly proximate the distal end of the tubular shaft. The basket assembly comprises a single unitary structure that includes a plurality of linear spines formed from a single piece of material and one or more electrodes coupled to each of the spines, each electrode defining a lumen through the electrode so that a spine extends through the lumen of each of the one or more electrodes. The spines converge at a central spine intersection at a distal end of the basket assembly. The central spine intersection includes one or more cutouts that allows for bending of the spines. Each spine comprises a respective end connected to the distal end of the tubular shaft.

## Description

### FIELD

The present invention relates generally to medical devices, and in particular catheters with electrodes, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that generally reduces thermal risks associated with RF ablation but may present tissue damage due to the very low temperature nature of such devices. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket. Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. What is needed, therefore, are devices and methods of forming an improved basket assembly that can help to reduce the time required for manufacturing the basket assembly, devices and methods for attaching electrodes to the spines, and alternative catheter geometries in general.

### SUMMARY

There is provided, in accordance with an embodiment of the present disclosure, a medical probe comprising an expandable basket assembly extending along a longitudinal axis. The basket assembly can comprise a plurality of spines, a polymer tube, and one or more electrodes. The plurality of spines can be disposed about the longitudinal axis from a first end to a second end. The polymer tube can be coupled to a spine in the plurality of spines such that the respective spine extends through the polymer tube. The one or more electrodes can be disposed on the polymer tube. Each electrode can comprise a substrate and catalytic material coating at least a portion of the substrate. The catalytic material can be selected from the group consisting of platinum (Pt), zirconium (Zr), hafnium (Hf), ruthenium (Ru), rhodium (Rh), palladium, (Pd), osmium (Os), iridium (Ir), gold (Au), Fe-N-C materials, and combinations thereof.

In any of the embodiments disclosed herein, the catalytic material can comprise platinum (Pt).

In any of the embodiments disclosed herein, each of the one or more electrodes can further comprise a conductive layer disposed between at least a portion of the polymer tube and the catalytic material coating.

In any of the embodiments disclosed herein, the substrate can comprise at least one of gold and stainless steel.

In any of the embodiments disclosed herein, the polymer tube can comprise a polymer selected from thermoset or thermoplastic polymer.

In any of the embodiments disclosed herein, the plurality of spines can be formed from a planar sheet of material.

In any of the embodiments disclosed herein, the planar sheet of material can comprise nitinol.

In any of the embodiments disclosed herein, the planar sheet of material can comprise cobalt chromium.

In any of the embodiments disclosed herein, the medical probe can further comprise a tubular shaft including a proximal end and a distal end, the tubular shaft can extend along the longitudinal axis, and the expandable basket can be disposed at the distal end of the tubular shaft.

In any of the embodiments disclosed herein, each spine can include a respective proximal end connected to the distal end of the tubular shaft.

In any of the embodiments disclosed herein, a distal end of the spines can converge at a central spine intersection proximately coincident with the longitudinal axis.

In any of the embodiments disclosed herein, the central spine intersection can include one or more cutouts that allow for bending of the spines.

In any of the embodiments disclosed herein, the central spine intersection can be positioned on a longitudinal axis at a distal end of the basket assembly.

In any of the embodiments disclosed herein, the plurality of spines can extend from the central spine intersection in an equiangular pattern such that respective angles between respectively adjacent spines are approximately equal.

In any of the embodiments disclosed herein, the plurality of spines can comprise three to ten spines.

In any of the embodiments disclosed herein, the plurality of spines can comprise exactly six spines.

In any of the embodiments disclosed herein, the expandable basket assembly can comprise a spherical shape.

In any of the embodiments disclosed herein, the expandable basket assembly can comprise an oblate-spheroid shape.

In any of the embodiments disclosed herein, the expandable basket assembly can comprise at least one discrete cutout located proximate the central spine intersection.

In any of the embodiments disclosed herein, the one or more electrodes can be configured to deliver electrical pulses for irreversible electroporation, and the pulses can include a peak voltage of at least 900 volts (V).

There is provided, in accordance with an embodiment of the present disclosure, a medical probe comprising a tubular shaft, an expandable basket assembly, and one or more electrodes. The tubular shaft can include a proximal end and a distal end. The tubular shaft can extend along a longitudinal axis. The expandable basket assembly can be disposed proximate the distal end of the tubular shaft. The basket assembly can comprise a plurality of spines. The one or more electrodes can be coupled to each of the spines. Each electrode can comprise a catalytic material selected from the group consisting of platinum (Pt), zirconium (Zr), hafnium (Hf) ruthenium (Ru), rhodium (Rh), palladium, (Pd), osmium (Os), iridium (Ir), gold (Au), and combinations thereof.

In any of the embodiments disclosed herein, the catalytic material can coat at least a portion of an exterior surface of the electrode.

In any of the embodiments disclosed herein, the catalytic material can coat an entirety of an exterior surface of the electrode.

In any of the embodiments disclosed herein, each of the plurality of spines can comprise a polymer tube defining a lumen so that the respective spine extends through the lumen, such that the one or more electrodes can be coupled to an outer surface of the polymer tube of each of the spines.

### BRIEF DESCRIPTION OF THE DRAWINGS

FIG. 1 is a schematic pictorial illustration of a medical system including a medical probe whose distal end includes a basket assembly with electrodes, in accordance with some embodiments of the disclosed technology;
FIG. 2 is a perspective view of a medical probe in an expanded form, in accordance with some embodiments of the disclosed technology;
FIG. 3 is a schematic pictorial illustration showing a perspective view of a spine with a polymer tube and electrode, in accordance with some embodiments of the disclosed technology;
FIG. 4A is a schematic pictorial illustration showing a cross-sectional view of a spine of a medical probe looking in a direction perpendicular to the longitudinal axis, in accordance with some embodiments of the disclosed technology;
FIG. 4B is a schematic pictorial illustration showing a cross-sectional view of a spine of a medical probe looking in a direction parallel to the longitudinal axis, in accordance with some embodiments of the disclosed technology;
FIGs. 4C-D are schematic pictorial illustrations showing cross-sectional views of a spine of a medical probe looking in a direction perpendicular to the longitudinal axis, in accordance with some embodiments of the disclosed technology;
FIGs. 5A-C are images of a spine of a medical probe, in accordance with some embodiments of the disclosed technology;
FIG. 6 is a schematic pictorial illustration of a central spine intersection, in accordance with some embodiments of the disclosed technology;
FIGs. 7A-J are schematic pictorial illustrations showing a perspective view of various example electrodes, in accordance with some embodiments of the disclosed technology;
FIGs. 8A-B are schematic pictorial illustrations showing various insulative jackets of a given medical device in accordance with some embodiments of the disclosed technology;
FIGs. 9-10 are flow charts of methods of constructing a medical probe, in accordance with some embodiments of the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including , but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, methods or uses and devices which utilize end effectors including electrodes affixed to spines. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

To deliver pulsed field ablation (PFA) in an IRE (irreversible electroporation) procedure, electrodes should contact the tissue being ablated with a sufficiently large surface area. As described hereinbelow, the medical probe includes a tubular shaft including proximal and distal ends, and a basket assembly at the distal end of the tubular shaft. In one example, the basket assembly can include a plurality of linear spines formed from a planar sheet of material and one or more electrodes coupled to each of the spines. The plurality of linear spines can converge at a central spine intersection including one or more cutouts. The cutouts can allow for bending of each spine such that the spines form an approximately spherical or oblate-spheroid basket assembly. It is noted that the cutouts (in various configurations described and illustrated in the specification) allows the basket to be compressed into a much smaller form factor when undeployed (or undergoing a retraction into a delivery sheath) without buckling or plastic deformation.

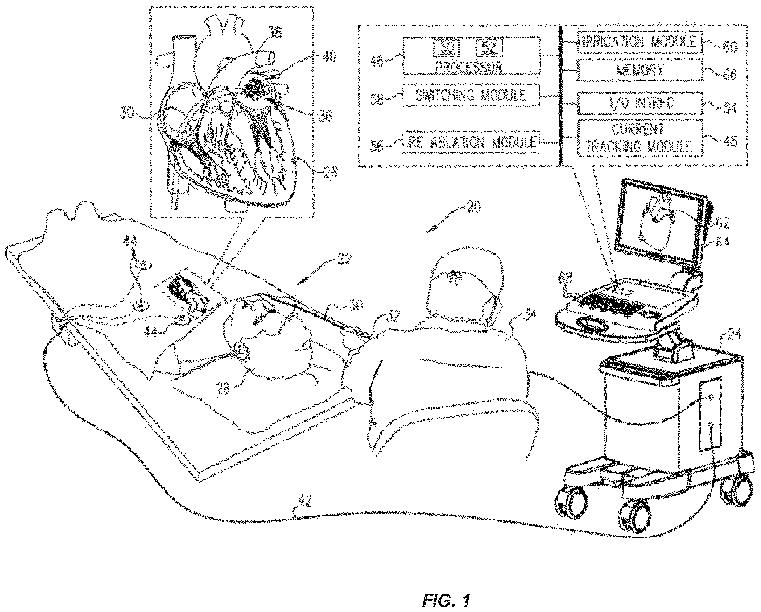

FIG. 1 is a schematic, pictorial illustration of a medical system 20 including a medical probe 22 and a control console 24, in accordance with an embodiment of the present invention. Medical system 20 may be based, for example, on the CARTO^{®} system, produced by Biosense Webster Inc. of 31 Technology Drive, Suite 200, Irvine, CA 92618 USA. In embodiments described hereinbelow, medical probe 22 can be used for diagnostic or therapeutic treatment, such as for performing ablation procedures in a heart 26 of a patient 28. Alternatively, medical probe 22 may be used, mutatis mutandis, for other therapeutic and/or diagnostic purposes in the heart or in other body organs.

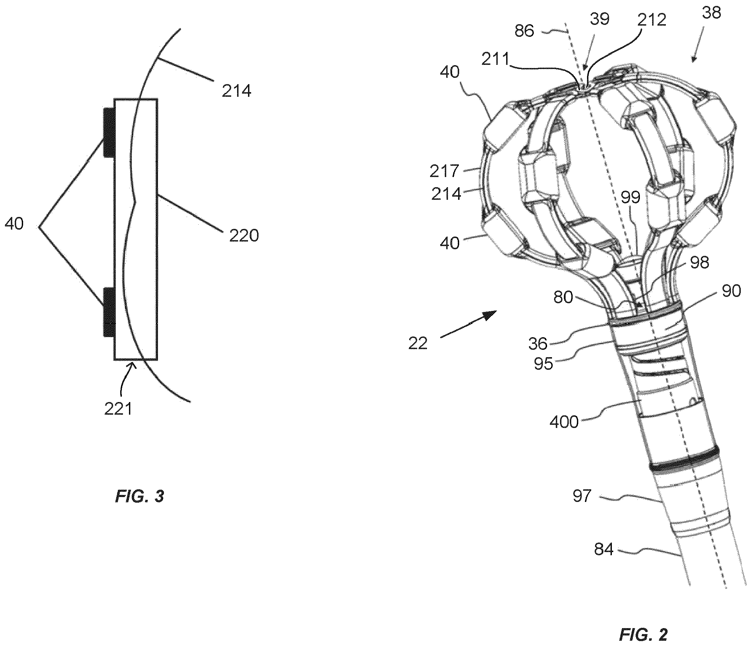

Medical probe 22 includes a flexible insertion tube 30 and a handle 32 coupled to a proximal end of the tubular shaft. During a medical procedure, a medical professional 34 can insert probe 22 through the vascular system of patient 28 so that a distal end 36 of the medical probe enters a body cavity such as a chamber of heart 26. Upon distal end 36 entering the chamber of heart 26, medical professional 34 can deploy a basket assembly 38 approximate a distal end 36 of the medical probe 22. Basket assembly 38 can include a plurality of electrodes 40 affixed to a plurality of spines 214, as described in the description referencing FIGs. 2-3 hereinbelow. To start performing a medical procedure such as irreversible electroporation (IRE) ablation, medical professional 34 can manipulate handle 32 to position distal end 36 so that electrodes 40 engage cardiac tissue at a desired location or locations. Upon positioning the distal end 36 so that electrodes 40 engages cardiac tissue, the medical professional 34 can activate the medical probe 22 such that electrical pulses are delivered by the electrodes 40 to perform the IRE ablation.

The medical probe 22 can include a guide sheath and a therapeutic catheter, wherein the guide sheath includes the flexible insertion tube 30 and the handle 32 and the therapeutic catheter includes the basket assembly 38, electrodes 40, and a tubular shaft 84 (see FIGs. 2 through 4). The therapeutic catheter is translated through the guide sheath so that the basket assembly 38 is positioned in the heart 26. The distal end 36 of the medical probe 22 corresponds to a distal end of the guide sheath when the basket assembly 38 is contained within the flexible insertion tube 30, and the distal end 36 of the medical probe 22 corresponds to a distal end of the basket assembly 38 when the basket assembly 38 is extended from the distal end of the guide sheath. The medical probe 22 can be alternatively configured to include a second handle on the therapeutic catheter and other features as understood by a person skilled in the pertinent art.

In the configuration shown in FIG. 1, control console 24 is connected, by a cable 42, to body surface electrodes, which typically include adhesive skin patches 44 that are affixed to patient 28. Control console 24 includes a processor 46 that, in conjunction with a tracking module 48, determines location coordinates of distal end 36 inside heart 26. Location coordinates can be determined based on electromagnetic position sensor output signals provided from the distal portion of the catheter when in the presence of a generated magnetic field. Location coordinates can additionally, or alternatively be based on impedances and/or currents measured between adhesive skin patches 44 and electrodes 40 that are affixed to basket assembly 38. In addition to being used as location sensors during a medical procedure, electrodes 40 may perform other tasks such as ablating tissue in the heart.

As described hereinabove, in conjunction with tracking module 48, processor 46 may determine location coordinates of distal end 36 inside heart 26 based on impedances and/or currents measured between adhesive skin patches 44 and electrodes 40. Such a determination is typically after a calibration process relating the impedances or currents to known locations of the distal end has been performed. While embodiments presented herein describe electrodes 40 that are preferably configured to deliver IRE ablation energy to tissue in heart 26, configuring electrodes 40 to deliver any other type of ablation energy to tissue in any body cavity is considered to be within the spirit and scope of the present invention. Furthermore, although described in the context of being electrodes 40 that are configured to deliver IRE ablation energy to tissue in the heart 26, one skilled in the art will appreciate that the disclosed technology can be applicable to electrodes used for mapping and/or determining various characteristics of an organ or other part of the patient's 28 body.

Processor 46 may include real-time noise reduction circuitry 50 typically configured as a field programmable gate array (FPGA), followed by an analog-to-digital (A/D) signal conversion integrated circuit 52. The processor can be programmed to perform one or more algorithms and uses circuitry 50 and circuit 52 as well as features of modules to enable the medical professional 34 to perform the IRE ablation procedure.

Control console 24 also includes an input/output (I/O) communications interface 54 that enables control console 24 to transfer signals from, and/or transfer signals to electrodes 40 and adhesive skin patches 44. In the configuration shown in FIG. 1, control console 24 additionally includes an IRE ablation module 56 and a switching module 58.

IRE ablation module 56 is configured to generate IRE pulses including peak power in the range of tens of kilowatts. In some examples, the electrodes 40 are configured to deliver electrical pulses including a peak voltage of at least 900 volts (V). The medical system 20 performs IRE ablation by delivering IRE pulses to electrodes 40. Preferably, the medical system 20 delivers biphasic pulses between electrodes 40 on the spine. Additionally, or alternatively, the medical system 20 delivers monophasic pulses between at least one of the electrodes 40 and a skin patch.

In order to dissipate the heat and to improve the efficiency of the ablation process, system 20 supplies irrigation fluid (e.g., a saline solution) to distal end 36 and to the electrodes 40 via a channel (not shown) in tubular shaft 84 (see FIG. 2). Additionally, or alternatively, irrigation fluid can be supplied through the flexible insertion tube 30. Control console 24 includes an irrigation module 60 to monitor and control irrigation parameters, such as the pressure and the temperature of the irrigation fluid. It is noted that while the preference for the exemplary embodiments of the medical probe is for IRE or PFA, it is within the scope of the present invention to also use the medical probe separately only for RF ablation (unipolar mode with an external grounding electrode or bipolar mode) or in combination with IRE and RF ablations sequentially (certain electrodes in IRE mode and other electrodes in RF mode) or simultaneously (groups of electrodes in IRE mode and other electrodes in RF mode).

Based on signals received from electrodes 40 and/or adhesive skin patches 44, processor 46 can generate an electroanatomical map 62 that shows the location of distal end 36 in the patient's body. During the procedure, processor 46 can present map 62 to medical professional 34 on a display 64, and store data representing the electroanatomical map in a memory 66. Memory 66 may include any suitable volatile and/or non-volatile memory, such as random-access memory or a hard disk drive.

In some embodiments, medical professional 34 can manipulate map 62 using one or more input devices 68. In alternative embodiments, display 64 may include a touchscreen that can be configured to accept inputs from medical professional 34, in addition to presenting map 62.

FIG. 2 is an illustration showing a perspective view of a medical probe 22 including a basket assembly 38 in an expanded form when unconstrained, such as by being advanced out of an insertion tube lumen 80 at a distal end 36 of an insertion tube 30. Probe 22 may include a contact force sensor assembly 400 to determine contact force of the spines against cardiac tissues. It should be noted that the medical probe 22 illustrated in FIG. 2 lacks the guide sheath illustrated in FIG. 1. In the expanded form shown in FIG. 2, spines 214 bow radially outwardly and in the collapsed form not shown the spines are arranged generally along a longitudinal axis 86 of insertion tube 30. For simplicity, only a single spine 214 has been marked with a reference number, but one of skill in the art will appreciate that the basket assembly 38 can include one or more spines 214 as illustrated in Fig. 2. Similarly, only electrodes 40 and an insulative sleeve 217 on the single spine 214 are marked with references numbers, but one of skill in the art will appreciate that the basket assembly 38 can include one or more electrodes 40 and one or more insulative sleeves depending on the particular configuration. A plurality of electrically insulative sleeves 217 can be provided so that each jacket can be disposed between a respective spine 214 of the plurality of spines 214 and a respective electrode 40 of the plurality of electrodes 40, thereby electrically isolating the plurality of electrodes 40 from the plurality of spines 214.

As shown in FIG. 2, basket assembly 38 includes a plurality of flexible spines 214 that are formed at the end of a tubular shaft 84 and are connected at both ends. During a medical procedure, medical professional 34 can deploy basket assembly 38 by extending tubular shaft 84 from insertion tube 30 causing basket assembly 38 to exit insertion tube 30 and transition to the expanded form. Spines 214 can have elliptical (e.g., circular), or rectangular cross-sections that may appear to be flat cross-sections, and include a flexible, resilient material (e.g., a shape-memory alloy such as nickel-titanium, also known as Nitinol) forming a strut as will be described in greater detail herein. As shown in FIG. 2, basket assembly 38 has a proximal portion with a distal end 39. The medical probe 22 can include a spine retention hub 90 that extends longitudinally from a distal end of tubular shaft 84 towards distal end 39 of basket assembly 38. As described supra, control console 24 includes irrigation module 60 that delivers irrigation fluid to basket assembly 38 through tubular shaft 84.

As shown in FIG. 2, the plurality of flexible linear spines 214 converge at a central spine intersection 211 that is also disposed on a longitudinal axis 86 defined by the spines 214. In some examples central spine intersection 211 can include one or more cutouts 212 that allow for bending of the spines 214 when each spine respective attachment end 216 is connected to the spine retention hub 90. Additionally, certain examples can be that the central spine intersection 211 links individual spines 214, each spine 214 linked to the intersection 211 separately.

As shown herein, electrodes 40 positioned on spines 214 of basket assembly 38 can be configured to deliver ablation energy RF and/or IRE to tissue in heart 26. Additionally, or alternatively, the electrodes 40 can also be used to determine the location of basket assembly 38 and/or to measure a physiological property such as local surface electrical potentials at respective locations on tissue in heart 26. The electrodes 40 can be biased such that a greater portion of the one or more electrodes 40 face outwardly from basket assembly 38 such that the one or more electrodes 40 deliver a greater amount of electrical energy outwardly away from the basket assembly 38 i.e., toward the heart 26 tissue than inwardly.

As described supra, control console 24 includes irrigation module 60 that delivers irrigation fluid to distal end 36. The multiple irrigation openings 98 can be angled to spray or otherwise disperse of the irrigation fluid to either a given electrode 40 or to tissue in heart 26. Since electrodes 40 do not include irrigation openings that deliver irrigation fluid, the configuration described hereinabove enables heat to be transferred from the tissue (i.e., during an ablation procedure) to the portion of the electrodes on the inner side of the spines 214, and the electrodes 40 can be cooled by aiming the irrigation fluid, via irrigation openings 98, at the portion of the electrodes 40 on the inner side of the spines 214. Spine retention hub electrode 99 disposed at a distal end of retention hub 90 can be used in combination with electrodes 40 on the spines 214, or alternatively, can be used independently from electrodes 40 for reference mapping or ablation.

The medical probe 22 can further include a contact force sensor assembly 400 that can be disposed in a contact force sensor assembly sleeve 95. The contact force sensor assembly sleeve 95 can be coupled to a proximal coupler 97 that can be coupled to the tubular shaft 84.

When the basket assembly 38 is deployed, the spines 214 define a three-dimensional shape including the profile. The basket assembly 38 can be approximately spheroid including an approximately circular profile. The basket assembly 38 can have an approximately oblate-spheroid shape including an approximately elliptical profile. Although not every variation of shape is shown or described herein, one skilled in the art will appreciate that spines 214 can be further configured to form other various shapes as would be suitable for the particular application.

By including spines 214 configured to form various shapes when in the expanded form, basket assembly 38 can be configured to position the various electrodes 40 attached to spines 214 at various locations, with each location being nearer or farther from the distal end of tubular shaft 84. In addition, each spine 214 may have an elliptical (e.g., circular) or rectangular (that may appear to be flat) cross-section, and include a flexible, resilient material (e.g., a shape-memory alloy such as nickel-titanium (also known as Nitinol), cobalt chromium, or any other suitable material).

In some embodiments, as shown in FIG. 3, at least a portion of each spine 214 can extend through a lumen 221 defined by a polymer tube 220. The polymer tube can comprise many different polymer materials known in the art, including, but not limited to, thermoset polymers and thermoplastic polymers. The one or more electrodes 40 can then be disposed on at least a portion of a surface of the polymer tube, ss shown in FIG. 3. The use of a polymer tube can be helpful for attaching the electrode 40 to the spine 214 during the assembly process, as it can be easier to insert the spine 214 through the lumen 221 of the polymer tube 220 than through a lumen of an electrode 40. In some embodiments, as shown in FIG. 3, multiple electrodes 40 can be disposed on the surface of a single polymer tube 220. In some embodiments, however, a single electrode 40 can be disposed on the surface of a single polymer tube 220. In such embodiments, each spine 214 can have one or more polymer tubes 220 with corresponding electrodes 40.

Examples of materials ideally suited for forming electrodes 40 include gold, platinum, palladium, ruthenium, rhodium, osmium, iridium, zirconium, hafnium, stainless steel, Fe-N-C compounds, and combinations thereof. These materials also have high thermal conductivity which allows the minimal heat generated on the tissue i.e., by the ablation energy delivered to the tissue to be conducted through the electrodes to the back side of the electrodes i.e., the portions of the electrodes on the inner sides of the spines, and then to the blood pool in heart 26.

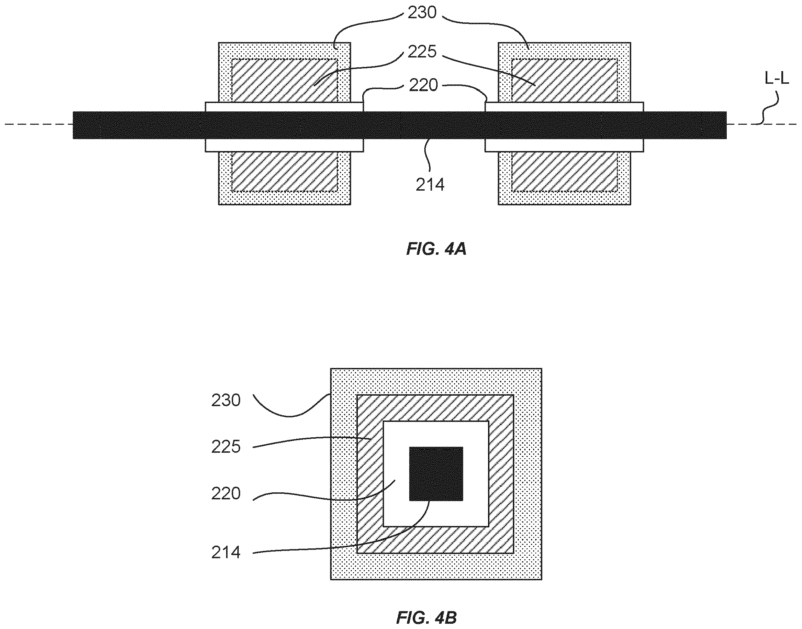

Turning to FIGs. 4A-D, in some embodiments, the electrodes 40 can comprise a conductive substrate 225 and a catalytic material 230, which is a different material than the substrate 225, coating at least a portion of the substrate 225. The catalytic material 230 can be many catalytic materials known in the art, including, but not limited to, platinum (Pt), ruthenium (Ru), rhodium (Rh), palladium, (Pd), osmium (Os), iridium (Ir) zirconium (Zr), hafnium (Hf), FeN-C compounds, and combinations thereof. In some embodiments, the catalytic material 230 can coat only a portion of an outer surface of the substrate 225. In some embodiments, as shown in FIGs. 4A-B, the catalytic material 230 can coat an entirety of the outer surface of the substrate 225.

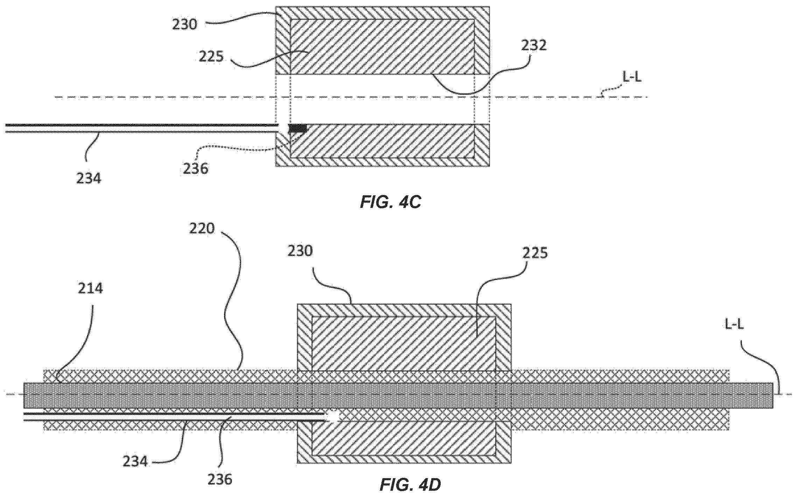

FIGs. 4A, 4C, and 4D illustrate cross-sectional views of a spine 214 looking in a direction perpendicular to the longitudinal axis L-L, while FIG. 4B illustrates a cross-sectional view of a spine 214 looking in a direction parallel to the longitudinal axis. A spine 214 can extend through the polymer tube 220 as discussed above. A substrate 225 of an electrode 40 can be disposed on an outer surface of the polymer tube 220. As shown in FIG. 4C, the substrate 225 can define an opening 232 extending along the longitudinal axis L-L. The polymer tube 220 can be disposed within the opening 232. A catalytic material 230 can coat the outer surface of the substrate 225.

As shown in FIGs. 4C-D, a lumen 234 can extend along a length of the polymer tube 220 and/or spine 214 having a conductive wire 236 disposed therein. The wire 236 can be coupled to the substrate 225 of the electrode 40, as shown in FIG. 4C, to provide electrical current to the electrode 40.

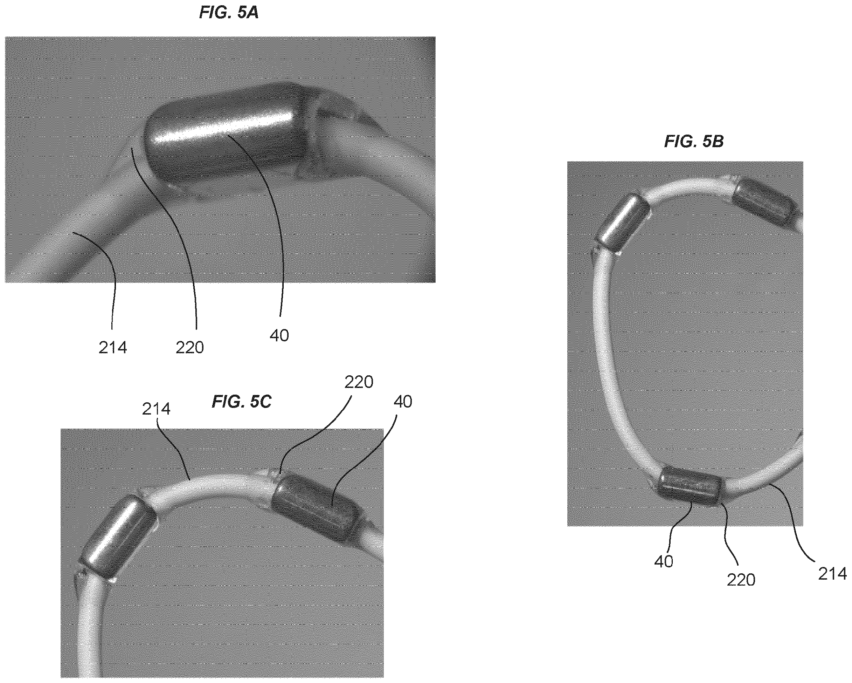

FIGs. 5A-C provide photographic images illustrating a spine 214 extending through the lumen of a polymer tube 220, in which an electrode 40 is disposed on an outer surface of the polymer tube 220.

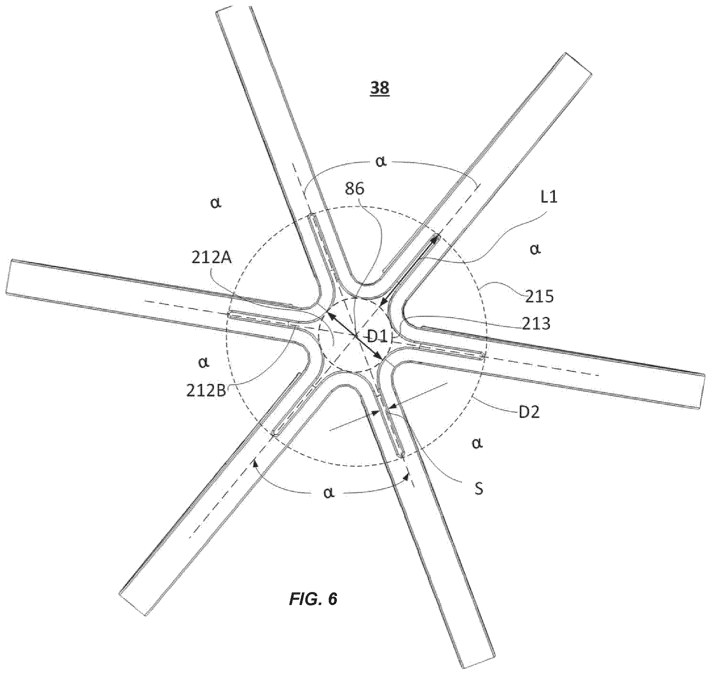

FIG. 6 is a schematic pictorial illustration showing a top-down view of basket assembly 38, showing an example of one or more cutouts 212 on central spine intersection 211. The spines 214 can be formed from a single sheet of planar material to form a generally star shape. In other words, spines 214 can be formed from the single sheet of planar material such that the spines 214 converge toward a central intersection 211. The intersection 211 can be a solid piece of material or include one or more cutouts 212. Basket assembly 38 can include a number of spines 214 ranging from about four to about ten spines from a single sheet of planar material.

In FIG. 6, the distal end of the basket assembly 38 has an open cutout 212 which is a combination of a central opening 212A (substantially approximated by a virtual circle 213 with diameter D1) and the groove 212B for each spine (giving a total of six grooves 212B). Each spine is disposed generally equiangularly about the longitudinal axis of a predetermined angle a between any two spines. Each groove 212B has groove width S that extend approximately a length L1 from the circumference of virtual circle D1 so that a virtual circle 215 with diameter D2 is contiguous to the grooves 212B. The second virtual circle 215 has a diameter D2 approximately 3.6 times that of the diameter D1 of the first virtual circle 213. In one embodiment, the cut-out 212 (represented by center cut out 212A and open grooves 212B) has a negative area of about 1.9 mm-squared with the diameter of the virtual circle 213 of about 1.1 mm and the virtual circle 215 of about 4mm such that each open groove 212B has a width S of about 0.08mm extending for about 1.5mm from the virtual circle 213 so that the negative area defined by all the cut-outs in this design includes approximately 1.9mm-squared.

As will be appreciated by one skilled in the art with the benefit of this disclosure, basket assembly 38 shown in FIGs. 2 including spines 214 formed from a single sheet of planar material and converging at a central intersection is offered merely for illustrative purposes and the disclosed technology can be applicable to other configurations of basket assemblies 38. For example, the described configuration of the basket spine assemblies can be obtained via laser cutting a nitinol tube and heat treating the spines from the tubular stock into substantially the planar form shown herein. As well, the disclosed technology can be applicable to basket assemblies 38 formed from a single spine 214 or multiple spines 214 with each spine 214 being attached at both ends. In other examples, the basket assembly 38 can include a central hub connecting the multiple spines 214 together at a distal end 39 of the basket assembly 38. In yet other examples, the basket assembly 38 can include a single spine 214 configured to form a spiral, multiple spines 214 configured to form a spiral, multiple spines 214 configured to form a tripod or multiple tripods, or any other shape of basket assembly 38. Thus, although FIG. 2 illustrates a specific configuration of basket assembly 38, the disclosed technology should not be construed as so limited.

In the exemplary embodiments shown herein, the spines width W may have a nominal width of approximately 0.6 mm and can be as low as 0.2 mm or as large as 1.5 mm. The thickness of each spine can be nominally 0.09 mm and can vary from 0.05mm to 0.2mm. It should be noted that these values for width and thickness can vary depending on the stiffness desired.

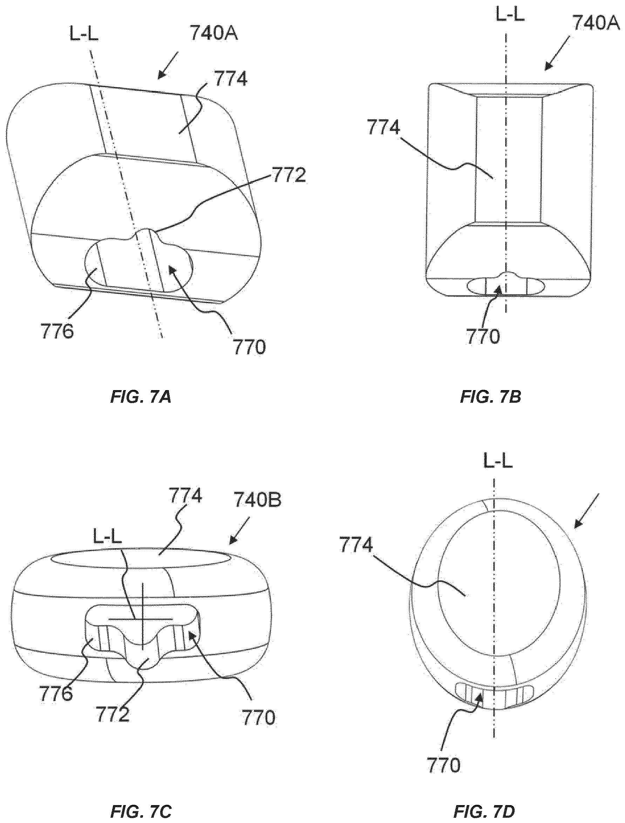

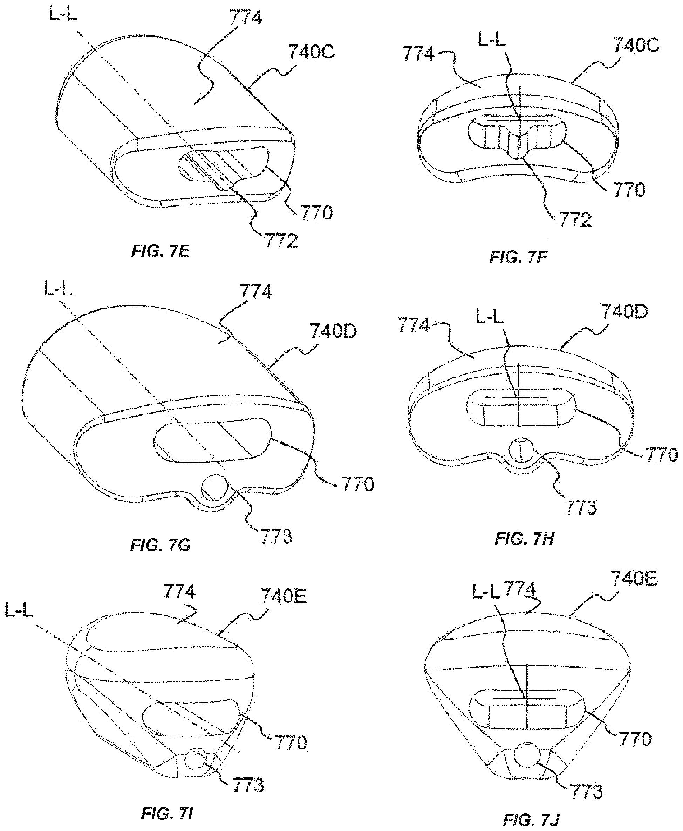

Turning to FIGs. 7A through 7J, electrode 40 can have a variety of cross-sectional shapes, curvatures, lengths, lumen number and lumen shape as provided as examples in electrodes 740A-740E. The electrodes 740A-740E are offered to illustrate various configurations of electrodes 40 that can be used with the medical device 22 but should not be construed as limiting. One skilled in the art will appreciate that various other configurations of electrodes 40 can be used with the disclosed technology without departing from the scope of this disclosure.

Each electrode 740A-740E can have an outer surface 774 facing outwardly from electrode 740 and an inner surface 776 facing inwardly toward electrode 740. Stated otherwise, outwardly with respect to a longitudinal axis L-L and facing the tissue or inward facing away from the tissue. At least one lumen 770 is formed through electrode 740. The lumen 770 can be sized and configured to receive a spine 214 such that spine 214 can pass through electrode 740. Lumen 770 can be a symmetric opening through electrode 740A-740E and can be disposed offset with respect to a longitudinal axis L-L of the respective electrode. In other examples, lumen 770 can pass through electrode 740 in a generally transverse direction with respect to the longitudinal axis L-L of the respective electrode. Furthermore, lumen 770 can be positioned in electrode 740 nearer a bottom surface, nearer a top surface, or nearer a middle of electrode 740 depending on the particular configuration. In FIGs. 7A, 7C, and 7E through 7J, the top surface (upper side) is oriented toward the top of the drawing, the bottom surface (lower side) is oriented toward the bottom of the drawing, and the middle is between the top surface and the bottom surface. In other words, each electrode 740A-740E can include a lumen 770 that is offset with respect to a centroid of the electrode 740A-740E.

In addition, as shown in FIGs. 7A through 7F, electrodes 740A-740C can have a wire relief 772 forming a recess or depression in electrode 740 adjacent lumen 770 for one or more wires to pass through lumen 770 along with a respective spine 214. Relief 772 can be sized to provide room for a wire of electrode 740 to pass through electrode 740 such that electrode 740 can be in electrical communication with the control console 24.

Alternatively, or in addition thereto, wires can pass through a wire lumen 773 as shown in example electrodes 740D and 740E in FIGs. 7G through 7J. Although not depicted, electrodes 40 may include both a wire relief 772 adjacent lumen 770 and wire lumen 773. Such electrode may permit additional wires to pass through the electrode body.

As shown in FIGs. 7A-7J, the electrodes 740A-740E can include various shapes depending on the application. For example, as illustrated in FIGs. 7A and 7B, the electrode 740A can have a substantially rectangular cuboid shape with rounded edges. In other examples, the electrode 740B can have a substantially ovoid shape (as illustrated in FIGs. 7C and 7D), the electrode 740C, 740D can have a contoured shape including a convex side and a concave side (as illustrated in FIGs. 7E through 7H), or the electrode 740E can have a contoured shape including substantially more material proximate an upper side than a lower side of the electrode 740E (as illustrated in FIGs. 7I and 7J). As will be appreciated by one of skill in the art, the various example electrodes 740A-740E shown in FIGs. 7A-7J, and described herein, are offered for illustrative purposes and should not be construed as limiting.

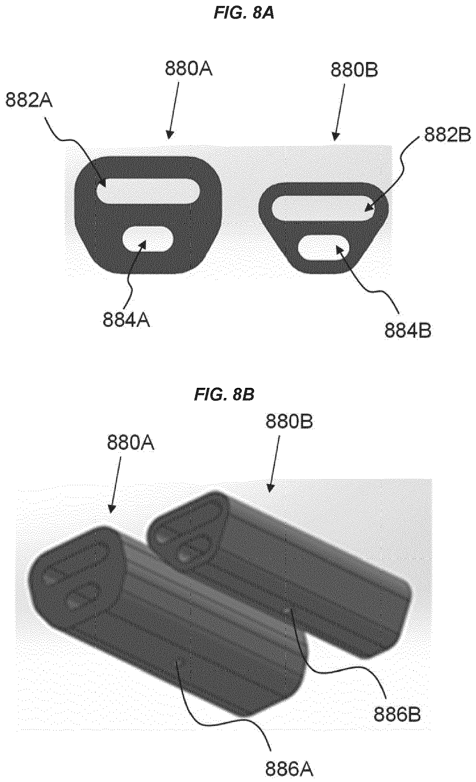

FIGs. 8A and 8B are schematic pictorial illustrations showing various insulative jackets 880A, 880B of a given medical device 22, in accordance with embodiments of the present invention. FIG. 8A is a front view while FIG. 8B is a perspective view of insulative jackets 880A, 880B. Insulative jackets 880A, 880B can be made from a biocompatible, electrically insulative material such as polyamide-polyether (Pebax) copolymers, polyethylene terephthalate (PET), urethanes, polyimide, parylene, silicone. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets 880A, 880B may also include one or more additives or fillers, such as, for example, polytetrafluoroethylene (PTFE), boron nitride, silicon nitride, silicon carbide, aluminum oxide, aluminum nitride, zinc oxide, and the like. Insulative jacket 880A, 880B can help to insulate a spine 214 and/or wires passing through insulative jacket 880A, 880B from electrode 40 to prevent arcing from electrode 40 to the spine 214 and/or mechanical abrasion of wires passing through insulative jacket 880A, 880B.

As illustrated in FIGs. 8A and 8B, insulative jackets 880A, 880B, can include a cross-sectional shape that is substantially trapezoidal. The insulative jacket may consist of a single lumen or multi-lumen configuration. Multi-lumen jackets may be configured such that the alloy frame and wires share a single lumen while the second lumen may be used for irrigation. The alloy frame and wires may occupy separate lumens, also, as described. The current embodiment does not utilize irrigated jackets. For these designs, the insulative jackets may be continuous (individual sleeves extending from proximal to distal end of each alloy frame strut), segmented (bridging between electrode gaps), or a combination of both. Furthermore, insulative jacket 880A, 880B can include a first lumen 882A, 882B and a second lumen 884A, 884B. First lumen 882A, 882B can be configured to receive spine 214 while second lumen 884A, 884B can be configured to receive a wire, or vice-versa. In other examples, first lumen 882A, 882B and second lumen 884A, 884B can each be configured to receive one or more wires that can be connected to one or more electrodes 40. Furthermore, as illustrated in FIG. 8B, insulative jacket 880A, 880B can include an aperture 886A, 886B through which a wire can be electrically connected to electrode 40. Although illustrated in FIG. 8B as being proximate a bottom of insulative jacket 880A, 880B, aperture 886A, 886B can be positioned proximate a top or side of insulative jacket 880A, 880B. Furthermore, insulative jacket 880A, 880B can include multiple apertures 886A, 886B with each aperture being disposed on the same side of insulative jacket (i.e., top, bottom, left, right) or on different sides of the insulative jacket depending on the application.

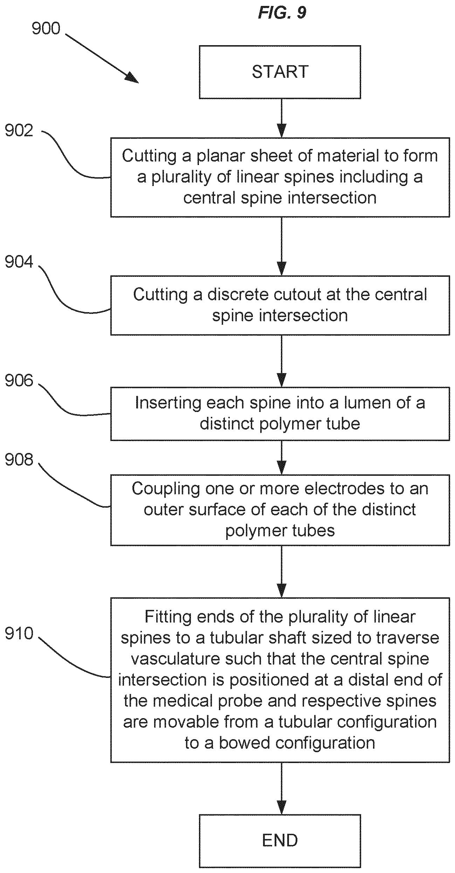

FIG. 9 is a flowchart illustrating a method 900 of manufacturing a basket assembly 38, in accordance with an embodiment of the present invention. Method 900 can include cutting 902 a planar sheet of material to form a plurality of linear spines 214 including a central spine intersection 211. The planar sheet of resilient material can include shape-memory alloy such as nickel-titanium (also known as Nitinol), cobalt chromium, or any other suitable material. Method 900 can include cutting 904 a discrete cutout 212 at the central spine intersection 211. As described supra, the discrete cutout 212 can be a single cutout or two or more cutouts. In addition, the one or more discrete cutouts can be cut in a pattern to extend along at least a portion of each spine. In some examples, steps 902 and 904 may occur as simultaneous steps or as a sequence of steps.

Method 900 can include inserting 904 each spine 214 into a lumen 221 of distinct polymer tube 220. In some embodiments, inserting 904 can include inserting a spine into a lumen of multiple polymer tubes 220. Method 900 can include coupling 908 one or more electrodes 40 to an outer surface of each of the distinct polymer tubes 220. In some embodiments, coupling 908 can include inserting the distinct polymer tube 220 through an opening 232 of the electrode 40. In some embodiment, coupling 908 can include placing an electrode 40 having an opening 232 over the polymer tube 220, such that the polymer tube 220 extends through the opening 232. In some embodiments, coupling 908 can occur prior to inserting 906. For example, an electrode 40 can be coupled to a polymer tube 220 by, e.g., inserting the polymer tube through an opening 232 of the electrode, and, then, a spine can be inserted into a lumen 221 of the polymer tube 220. In some embodiments, coupling 908 can occur after inserting 906. For example, a spine 214 can be inserted into a lumen 221 of a polymer tube 220, and, then, an electrode 40 can be coupled to the polymer tube 220 by, e.g., placing an electrode 40 having an opening 232 over the polymer tube 220, such that the polymer tube 220 extends through the opening 232. The electrodes can be positioned such that the electrodes are offset between electrodes on adjacent spines.

Method 900 can include fitting 910 ends of the plurality of linear spines to a tubular shaft sized to traverse vasculature such that the central spine intersection is positioned at a distal end of the medical probe and respective spines are movable from a tubular configuration to a bowed configuration. As will be appreciated by one of skill in the art including the benefit of this disclosure, fitting 910 an end of the spine into a tubular shaft can include attaching the spine 214 to a spine retention hub 90. Furthermore, the spine retention hub 90 and/or the spine 214 and the tubular shaft 84 can be inserted into a flexible insertion tube 30 to form the medical probe 22

In some examples, the method 900 can also include forming an approximately spheroid or oblate-spheroid shape with the linear spines. Method 900 can further include electrically connecting the wire 236 to the one or more electrodes.

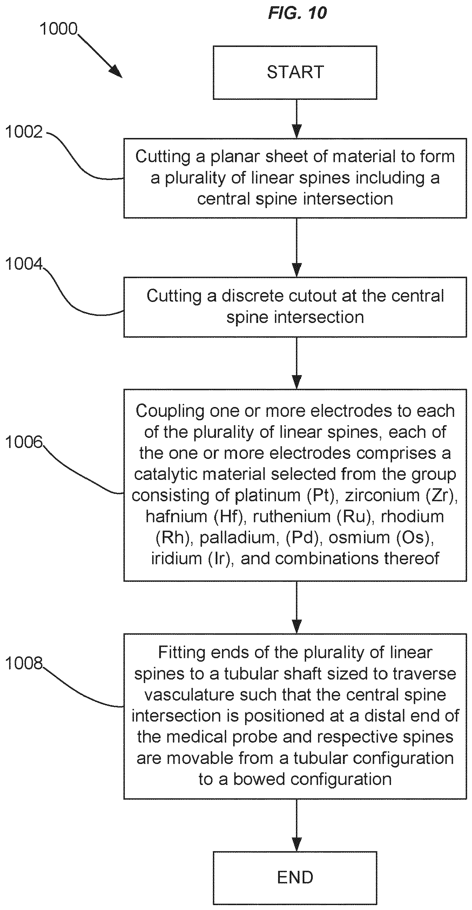

FIG. 10 is a flowchart illustrating a method 1000 of manufacturing a basket assembly 38, in accordance with an embodiment of the present invention. Method 1000 can include cutting 1002 a planar sheet of material to form a plurality of linear spines 214 including a central spine intersection 211. The planar sheet of resilient material can include shape-memory alloy such as nickel-titanium (also known as Nitinol), cobalt chromium, or any other suitable material. Method 1000 can include cutting 1004 a discrete cutout 212 at the central spine intersection 211. As described supra, the discrete cutout 212 can be a single cutout or two or more cutouts. In addition, the one or more discrete cutouts can be cut in a pattern to extend along at least a portion of each spine. In some examples, steps 1002 and 1004 may occur as simultaneous steps or as a sequence of steps.

Method 1000 can include coupling 1006 one or more electrodes 40 to each of the plurality of linear spines 214. Each of the one or more electrodes can comprise a catalytic material. The catalytic material can comprise platinum, zirconium, hafnium, ruthenium, rhodium, palladium, osmium, iridium, gold, and/or combinations thereof.

Method 1000 can include fitting 1008 ends of the plurality of linear spines to a tubular shaft sized to traverse vasculature such that the central spine intersection is positioned at a distal end of the medical probe and respective spines are movable from a tubular configuration to a bowed configuration. As will be appreciated by one of skill in the art including the benefit of this disclosure, fitting 1008 an end of the spine into a tubular shaft can include attaching the spine 214 to a spine retention hub 90. Furthermore, the spine retention hub 90 and/or the spine 214 and the tubular shaft 84 can be inserted into a flexible insertion tube 30 to form the medical probe 22

In some examples, the method 1000 can also include forming an approximately spheroid or oblate-spheroid shape with the linear spines. Method 1000 can further include electrically connecting the wire 236 to the one or more electrodes.

In some embodiments, various steps of method 900 and method 1000 can be combined to manufacture a basket assembly 38.

As will be appreciated by one skilled in the art, method 900 and method 1000 can include any of the various features of the disclosed technology described herein and can be varied depending on the particular configuration, such as starting with a tube rather than a planar sheet. Thus, methods 900 and 1000 should not be construed as limited to the particular steps and order of steps explicitly described herein. It is noted that while the preference for the exemplary embodiments of the medical probe is for IRE or PFA, it is within the scope of the present invention to also use the medical probe separately only for RF ablation (unipolar mode with an external grounding electrode or bipolar mode) or in combination with IRE and RF ablations sequentially (certain electrodes in IRE mode and other electrodes in RF mode) or simultaneously (groups of electrodes in IRE mode and other electrodes in RF mode).

Examples are described below which can be used separately or in various permutations with each other and such examples (in various permutations) may be used to define the scope of the invention.
**Example 1)** A medical probe is provided which may include a tubular shaft including a proximal end and a distal end. The tubular shaft can extend along a longitudinal axis. The medical probe can include an expandable basket assembly proximate the distal end of the tubular shaft. The basket assembly can include a single unitary structure that includes a plurality of linear spines formed from a planar sheet of material. The spines can converge at a central spine intersection. The central spine intersection can have one or more cutouts that allows for bending of the spines. Each spine can have a respective end connected to the distal end of the tubular shaft. The central spine intersection can be positioned on the longitudinal axis at a distal end of the basket assembly. The expandable basket assembly can include one or more electrodes coupled to each of the spines. Each electrode can define a lumen through the electrode so that a spine extends through the lumen of each of the one or more electrodes.
**Example 2)** The plurality of linear spines can extend from the central spine intersection in an equiangular pattern such that respective angles between respectively adjacent spines are approximately equal.
**Example 3)** The expandable basket assembly can include three to ten spines of the plurality of spines. The expandable basket assembly can include exactly six spines of the plurality of spines. The expandable basket assembly can be approximately spherical. The expandable basket assembly can be approximately oblate-spheroid.
**Example 4)** The electrodes can comprise a conductive substrate and a catalytic material coating at least a portion of the conductive substrate. The catalytic material can comprise platinum, zirconium, hafnium, ruthenium, rhodium, palladium, osmium, iridium, gold, and/or combinations thereof.
**Example 5)** The expandable basket assembly can include at least one discrete cutout located proximate the central spine intersection. The one or more cutouts can include a centrosymmetric pattern. The one or more cutouts can include an equiangular pattern. The one or more cutouts can extend along at least a portion of each spine.
**Example 6)** Each electrode can include a wire relief adjacent the lumen to allow for one or more wires to extend adjacent to the lumen. The electrode lumen can be disposed symmetrically about a longitudinal axis of the electrode. The one or more electrodes can be configured to deliver electrical pulses for irreversible electroporation. The electrical pulses can have a peak voltage of at least 900 volts (V).
**Example 7)** The medical probe can further include irrigation openings disposed proximate the distal end of the tubular shaft. The irrigation openings can be configured to deliver an irrigation fluid to the one or more electrodes.
**Example 8)** The medical probe can further include polymer tubes each disposed over the respective given spine and within an opening of the respective electrode. Each polymer tube can include a first lumen through which the respective given spine can extend and a second lumen through which an electrical wire can extend. The first and second lumens can be distinct from each other and each polymer tube can extend within the lumen of the respective electrode.
**Example 9)** The medical probe can further include a plurality of wires each electrically joined to a respective electrode of the one or more electrodes.
**Example 10)** The planar sheet of material can include nitinol. The planar sheet of material can include cobalt chromium.
**Example 11)** In some examples, an embodiment of the present invention provides a method of constructing a medical probe. The method can include cutting a planar sheet of material to form a plurality of linear spines including a central spine intersection, cutting a discrete cutout at the central spine intersection, inserting each spine into a lumen of a distinct polymer tube, coupling one or more electrodes to an outer surface of each of the distinct polymer tubes, and fitting ends of the plurality of linear spines to a tubular shaft sized to traverse vasculature such that the central spine intersection is positioned at a distal end of the medical probe and respective spines are movable from a tubular configuration to a bowed configuration.
**Example 12)** The method can further include cutting the plurality of linear spines from a pattern including longitudinal and transverse scores. The method can further include cutting at least two cutouts at the central spine intersection. The method can further include cutting a single discrete cutout at the central spine intersection. The method can further include cutting the single discrete cutout along at least a portion of each spine. The method can further include cutting each discrete cutout along at least a portion of each spine.
**Example 13)** The linear spines can form an approximately oblate-spheroid shape in the predetermined shape.
**Example 14)** The method can further include offsetting the electrodes between adjacent spines. Each electrode can include a relief adjacent the lumen to allow a wire to extend adjacent to the lumen. The wire can be electrically insulated from the spine.
**Example 15)** In some examples, an embodiment of the present invention provides a method of constructing a medical probe. The method can include cutting a planar sheet of material to form a plurality of linear spines including a central spine intersection, cutting a discrete cutout at the central spine intersection, inserting each spine into a lumen of a distinct polymer tube, coupling one or more electrodes to each of the plurality of linear spines, each of the one or more electrodes comprising a catalytic material selected from the group consisting of platinum, zirconium, hafnium, ruthenium, rhodium, palladium, osmium, iridium, gold, and combinations thereof, and fitting ends of the plurality of linear spines to a tubular shaft sized to traverse vasculature such that the central spine intersection is positioned at a distal end of the medical probe and respective spines are movable from a tubular configuration to a bowed configuration.

The disclosed technology described herein can be further understood according to the following clauses:
Clause 1: An electrode to deliver high-voltage direct current to induce electroporation in mammalian cells, comprising: a substrate extending along a longitudinal axis, the substrate defining an opening disposed around the longitudinal axis; and a catalytic material coating at least a portion of the substrate, the catalytic material being different from the substrate and selected from the group consisting of platinum (Pt), ruthenium (Ru), rhodium (Rh), palladium, (Pd), osmium (Os), iridium (Ir) zirconium (Zr), hafnium (Hf), gold (Au), Fe-N-C compounds, and combinations thereof.
Clause 2: The electrode of clause 1, in which the substrate comprises a conductive material.
Clause 3: The electrode of clause 1, in which the substrate comprises a non-conductive material
Clause 4: The electrode of clause 1, the catalytic material coating the entirety of the substrate.
Clause 5: The electrode according to clause 2, the conductive material comprises at least one of gold, Nitinol, and stainless steel.
Clause 6: The electrode according to any one of clauses 1-5, the catalytic material comprises a thickness of about 0.0003 inches.
Clause 7: The electrode according to any one of clauses 1-6, the high-voltage direct current comprises a peak voltage of between 900 and 2,000 volts (V).
Clause 8: A medical probe, comprising: an expandable basket assembly extending along a longitudinal axis, the basket assembly comprising: a plurality of spines disposed about the longitudinal axis from a first end to a second end; a polymer tube coupled to a spine in the plurality of spines such that the respective spine extends through the polymer tube; and one or more electrodes disposed on the polymer tube, each electrode comprising a substrate and catalytic material coating at least a portion of the substrate, the catalytic material selected from the group consisting of platinum (Pt), zirconium (Zr), hafnium (Hf) ruthenium (Ru), rhodium (Rh), palladium, (Pd), osmium (Os), iridium (Ir), gold (Au), Fe-N-C materials , and combinations thereof.
Clause 9: The medical probe of clause 8, the catalytic material comprises platinum (Pt).
Clause 10: The medical probe of clause 8, each of the one or more electrodes further comprising a conductive layer disposed between at least a portion of the polymer tube and the catalytic material coating.
Clause 11: The medical probe of clause 10, wherein the substrate comprises at least one of gold and stainless steel.
Clause 12: The medical probe according to any of clauses 8-11, the polymer tube comprises a polymer selected from thermoset or thermoplastic polymer.
Clause 13: The medical probe according to any one of clauses 8-12, the plurality of spines are formed from a planar sheet of material.
Clause 14: The medical probe according to clause 12, the planar sheet of material comprises nitinol.
Clause 15: The medical probe according to clause 12, the planar sheet of material comprises cobalt chromium.
Clause 16: The medical probe according to any one of clauses 8-15, further comprising a tubular shaft including a proximal end and a distal end, the tubular shaft extending along the longitudinal axis, wherein the expandable basket is disposed at the distal end of the tubular shaft.
Clause 17: The medical probe of clause 16, wherein each spine includes a respective proximal end connected to the distal end of the tubular shaft.
Clause 18: The medical probe according to clauses 16 or 17, wherein a distal end of the spines converge at a central spine intersection proximately coincident with the longitudinal axis.
Clause 19: The medical probe of clause 18, wherein the central spine intersection includes one or more cutouts that allow for bending of the spines.
Clause 20: The medical probe according to clauses 18 or 19, wherein the central spine intersection is positioned on a longitudinal axis at a distal end of the basket assembly.
Clause 21: The medical probe according to any one of clauses 18-20, wherein the plurality of spines extend from the central spine intersection in an equiangular pattern such that respective angles between respectively adjacent spines are approximately equal.
Clause 22: The medical probe according to any one of clauses 8-21, wherein the plurality of spines comprises three to ten spines.
Clause 23: The medical probe according to any one of clauses 8-22, wherein the plurality of spines comprises exactly six spines.
Clause 24: The medical probe according to any one of clauses 8-23, wherein the expandable basket assembly comprises a spherical shape.
Clause 25: The medical probe according to any one of clauses 8-24, wherein the expandable basket assembly comprises an oblate-spheroid shape.
Clause 26: The medical probe according to any one of clauses 8-25, wherein the expandable basket assembly comprises at least one discrete cutout located proximate the central spine intersection.
Clause 27: The medical probe according to any one of clauses 8-26, wherein the one or more electrodes are configured to deliver electrical pulses for irreversible electroporation, the pulses including a peak voltage of at least 900 volts (V).
Clause 28: A medical probe, comprising: a tubular shaft including a proximal end and a distal end, the tubular shaft extending along a longitudinal axis; an expandable basket assembly proximate the distal end of the tubular shaft, the basket assembly comprising a plurality of spines; and one or more electrodes coupled to each of the spines, each electrode comprising a catalytic material selected from the group consisting of platinum (Pt), zirconium (Zr), hafnium (Hf) ruthenium (Ru), rhodium (Rh), palladium, (Pd), osmium (Os), iridium (Ir), gold (Au), and combinations thereof.
Clause 29: The medical probe of clause 28, wherein the catalytic material coats at least a portion of an exterior surface of the electrode.
Clause 30: The medical probe of clause 28, wherein the catalytic material coats an entirety of an exterior surface of the electrode.
Clause 31: The medical probe according to any of clauses 28-30, wherein the catalytic material comprises platinum (Pt).
Clause 32: The medical probe according to any of clauses 28-31, wherein each of the plurality of spines comprises a polymer tube defining a lumen so that the respective spine extends through the lumen, such that the one or more electrodes are coupled to an outer surface of the polymer tube of each of the spines.
Clause 33: The medical probe according to clause 32, wherein the polymer comprises a polymer selected from thermoset or thermoplastic polymer.
Clause 34: The medical probe according to any one of clauses 28-33, wherein the plurality of spines are formed from a planar sheet of material.
Clause 35: The medical probe according to clause 34, wherein the planar sheet of material comprises nitinol.
Clause 36: The medical probe according to clause 34, wherein the planar sheet of material comprises cobalt chromium.
Clause 37: The medical probe according to any one of clauses 28-36, wherein each spine includes a respective proximal end connected to the distal end of the tubular shaft.
Clause 38: The medical probe according to any one of clauses 28-37, wherein a distal end of the spines converge at a central spine intersection.
Clause 39: The medical probe of clause 38, wherein the central spine intersection includes one or more cutouts that allow for bending of the spines.
Clause 40: The medical probe according to clauses 38 or 39, wherein the central spine intersection is positioned on the longitudinal axis at a distal end of the basket assembly.
Clause 41: The medical probe according to any one of clauses 38-40, wherein the plurality of spines extend from the central spine intersection in an equiangular pattern such that respective angles between respectively adjacent spines are approximately equal.
Clause 42: The medical probe according to any one of clauses 28-41, wherein the plurality of spines comprises four to ten spines.
Clause 43: The medical probe according to any one of clauses 28-42, wherein the plurality of spines comprises exactly six spines.
Clause 44: The medical probe according to any one of clauses 28-43, wherein the expandable basket assembly comprises a spherical shape.
Clause 45: The medical probe according to any one of clauses 28-45, wherein the expandable basket assembly comprises an oblate-spheroid shape.
Clause 46: The medical probe according to any one of clauses 28-45, wherein the expandable basket assembly comprises at least one discrete cutout located proximate the central spine intersection.
Clause 47: The medical probe according to any one of clauses 28-46, wherein the one or more electrodes are configured to deliver electrical pulses for irreversible electroporation, the pulses including a peak voltage of at least 900 volts (V).
Clause 48: A medical probe, comprising: a tubular shaft including a proximal end and a distal end, the tubular shaft extending along a longitudinal axis; an expandable basket assembly proximate the distal end of the tubular shaft, the basket assembly comprising a plurality of spines, each spine comprising a polymer tube defining a lumen so that the respective spine extends through the lumen; and one or more electrodes coupled to an outer surface of the polymer tube of each of the spines.
Clause 49: The medical probe of clause 48, wherein the polymer comprises a polymer selected from thermoset or thermoplastic polymer.
Clause 50: The medical probe according to clauses 48or 49, wherein each electrode comprises a catalytic material coating at least a portion of the polymer tube, the catalytic material selected from the group consisting of platinum (Pt), zirconium (Zr), hafnium (Hf) ruthenium (Ru), rhodium (Rh), palladium, (Pd), osmium (Os), iridium (Ir), gold (Au), and combinations thereof.
Clause 51: The medical probe of clause 50, wherein the catalytic material is platinum (Pt).
Clause 52: The medical probe according to any one of clauses 48-51, wherein the plurality of spines are formed from a planar sheet of material.
Clause 53: The medical probe according to clause 52, wherein the planar sheet of material comprises nitinol.
Clause 54: The medical probe according to clause 52, wherein the planar sheet of material comprises cobalt chromium.
Clause 55: The medical probe according to any one of clauses 48-54, wherein each spine includes a respective proximal end connected to the distal end of the tubular shaft.
Clause 56: The medical probe according to any one of clauses 48-55, wherein a distal end of the spines converge at a central spine intersection.
Clause 57: The medical probe of clause 56, wherein the central spine intersection includes one or more cutouts that allow for bending of the spines.
Clause 58: The medical probe according to clauses 56 or 57, wherein the central spine intersection is positioned on the longitudinal axis at a distal end of the basket assembly.
Clause 59: The medical probe according to any one of clauses 56-58, wherein the plurality of spines extend from the central spine intersection in an equiangular pattern such that respective angles between respectively adjacent spines are approximately equal.
Clause 60: The medical probe according to any one of clauses 48-59, wherein the plurality of spines comprises three to ten spines.
Clause 61: The medical probe according to any one of clauses 48-60, wherein the plurality of spines comprises exactly six spines.
Clause 62: The medical probe according to any one of clauses 48-61, wherein the expandable basket assembly comprises a spherical shape.
Clause 63: The medical probe according to any one of clauses 48-62, wherein the expandable basket assembly comprises an oblate-spheroid shape.
Clause 64: The medical probe according to any one of clauses 48-63, wherein the expandable basket assembly comprises at least one discrete cutout located proximate the central spine intersection.
Clause 65: The medical probe according to any one of clauses 48-64, wherein the one or more electrodes are configured to deliver electrical pulses for irreversible electroporation, the pulses including a peak voltage of at least 900 volts (V).
Clause 66: A method of constructing a medical probe, the method comprising: cutting a planar sheet of material to form a plurality of spines including a central spine intersection; cutting a discrete cutout at the central spine intersection; inserting each spine into a lumen of a distinct polymer tube; coupling one or more electrodes to an outer surface of each of the distinct polymer tubes; and fitting ends of the plurality of spines to a tubular shaft sized to traverse vasculature such that the central spine intersection is positioned at a distal end of the medical probe and respective spines are movable from a tubular configuration to a bowed configuration.
Clause 67: The method of clause 66, wherein the polymer tube comprises a polymer selected from thermoset or thermoplastic polymer.
Clause 68: The method according to clauses 66 or 67, wherein coupling the one or more electrodes to an outer surface of each of the distinct polymer tubes comprises electroplating the one or more electrodes to the outer surface of each of the distinct polymer tubes.
Clause 69: The method according to any one of clauses 66-68, wherein the one or more electrodes comprises a catalytic material selected from the group consisting of platinum (Pt), zirconium (Zr), hafnium (Hf) ruthenium (Ru), rhodium (Rh), palladium, (Pd), osmium (Os), iridium (Ir), gold (Au), and combinations thereof.
Clause 70: The method of clause 69, wherein the catalytic material is platinum (Pt).
Clause 71: The method according to any one of clauses 66-70, further comprising cutting the plurality of spines from a pattern comprising longitudinal and transverse scores.
Clause 72: The method according to any one of clauses 66-71, further comprising offsetting the one or more electrodes between adjacent spines.
Clause 73: The method according to any one of clauses 66-72, wherein the spines form an oblate-spheroid shape in the bowed configuration.
Clause 74: The method according to any one of clauses 66-72, wherein the spines form a spherical shape in the bowed configuration.
Clause 75: The method according to any one of clauses 66-74, wherein the one or more electrodes are configured to deliver electrical pulses for irreversible electroporation, the pulses including a voltage of at least 900 volts (V).
Clause 76: A method of constructing a medical probe, the method comprising: cutting a planar sheet of material to form a plurality of spines including a central spine intersection; cutting a discrete cutout at the central spine intersection; coupling one or more electrodes to each of the plurality of spines, each of the one or more electrodes comprises a catalytic material selected from the group consisting of platinum (Pt), zirconium (Zr), hafnium (Hf), ruthenium (Ru), rhodium (Rh), palladium, (Pd), osmium (Os), iridium (Ir), gold (Au), and combinations thereof; and fitting ends of the plurality of spines to a tubular shaft sized to traverse vasculature such that the central spine intersection is positioned at a distal end of the medical probe and respective spines are movable from a tubular configuration to a bowed configuration.
Clause 77: The method of clause 76, further comprising inserting the inserting each of the plurality of spines into a lumen of a distinct polymer tube, and wherein coupling the one or more electrodes to each of the plurality of spines comprises coupling the one or more electrodes to an outer surface of each of the distinct polymer tubes.
Clause 78: The method according to clauses 76 or 77, wherein coupling the one or more electrodes to the outer surface of each of the distinct polymer tubes comprises electroplating the one or more electrodes to the outer surface of each of the distinct polymer tubes.
Clause 79: The method according to any of clauses 76-78, wherein each of the polymer tubes comprises a polymer selected from thermoset or thermoplastic polymer.
Clause 80: The method according to any of clauses 76-79, wherein the catalytic material is platinum (Pt).
Clause 81: The method according to any one of clauses 76-80, further comprising cutting the plurality of spines from a pattern comprising longitudinal and transverse scores.
Clause 82: The method according to any one of clauses 76-81, further comprising offsetting the one or more electrodes between adjacent spines.
Clause 83: The method according to any one of clauses 76-82, wherein the spines form an oblate-spheroid shape in the bowed configuration.
Clause 84: The method according to any one of clauses 76-82, wherein the spines form a spherical shape in the bowed configuration.
Clause 85: The method according to any one of clauses 76-84, wherein the one or more electrodes are configured to deliver electrical pulses for irreversible electroporation, the pulses including a voltage of at least 900 volts (V).

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. An electrode to deliver high-voltage direct current to induce electroporation in mammalian cells, comprising:
a substrate extending along a longitudinal axis, the substrate defining an opening disposed around the longitudinal axis; and
a catalytic material coating at least a portion of the substrate, the catalytic material being different from the substrate and selected from the group consisting of platinum (Pt), ruthenium (Ru), rhodium (Rh), palladium, (Pd), osmium (Os), iridium (Ir) zirconium (Zr), hafnium (Hf), gold (Au), Fe-N-C compounds, and combinations thereof.

2. The electrode of claim 1, in which the substrate comprises a conductive material., optionally wherein the conductive material comprises at least one of gold, Nitinol, and stainless steel.

3. The electrode of claim 1, in which the substrate comprises a non-conductive material

4. The electrode of claim 1, the catalytic material coating the entirety of the substrate.

5. The electrode of claim 1, the catalytic material comprises a thickness of about 0.0003 inches.

6. The electrode of claim 1, the high-voltage direct current comprises a peak voltage of between 900 and 2,000 volts (V).

7. A medical probe, comprising:
an expandable basket assembly extending along a longitudinal axis, the basket assembly comprising:
a plurality of spines disposed about the longitudinal axis from a first end to a second end;
a polymer tube coupled to a spine in the plurality of spines such that the respective spine extends through the polymer tube; and
one or more electrodes disposed on the polymer tube, each electrode comprising a substrate and catalytic material coating at least a portion of the substrate, the catalytic material selected from the group consisting of platinum (Pt), zirconium (Zr), hafnium (Hf) ruthenium (Ru), rhodium (Rh), palladium, (Pd), osmium (Os), iridium (Ir), gold (Au), Fe-N-C materials , and combinations thereof.

8. The medical probe of claim 7, the catalytic material comprises platinum (Pt).

9. The medical probe of claim 7, each of the one or more electrodes further comprising a conductive layer disposed between at least a portion of the polymer tube and the catalytic material coating, optionally wherein the substrate comprises at least one of gold and stainless steel.

10. The medical probe of claim 7, the polymer tube comprises a polymer selected from thermoset or thermoplastic polymer.

11. The medical probe of claim 7, the plurality of spines are formed from a planar sheet of material.

12. The medical probe of claim 10, the planar sheet of material comprises nitinol, or cobalt chromium.

13. The medical probe of claim 7, further comprising a tubular shaft including a proximal end and a distal end, the tubular shaft extending along the longitudinal axis, wherein the expandable basket is disposed at the distal end of the tubular shaft.

14. The medical probe of claim 13, wherein each spine includes a respective proximal end connected to the distal end of the tubular shaft.

15. The medical probe of claim 13, wherein a distal end of the spines converge at a central spine intersection proximately coincident with the longitudinal axis, optionally wherein the central spine intersection includes one or more cutouts that allow for bending of the spines.

16. A medical probe, comprising:
a tubular shaft including a proximal end and a distal end, the tubular shaft extending along a longitudinal axis;
an expandable basket assembly proximate the distal end of the tubular shaft, the basket assembly comprising a plurality of spines; and
one or more electrodes coupled to each of the spines, each electrode comprising a catalytic material selected from the group consisting of platinum (Pt), zirconium (Zr), hafnium (Hf) ruthenium (Ru), rhodium (Rh), palladium, (Pd), osmium (Os), iridium (Ir), gold (Au), and combinations thereof.
